(19) Europäisches Patentamt
European Patent Office
Office européen des brevets

(11) Publication number: 0 118 982 A1

# (12) EUROPEAN PATENT APPLICATION

(21) Application number: 84300532.3

(22) Date of filing: 27.01.84

(51) Int. Cl.³: C 07 F 9/65
A 01 N 57/16, C 07 D 241/38

(30) Priority: 01.02.83 JP 15838/83
12.10.83 JP 190545/83

(43) Date of publication of application: 19.09.84 Bulletin 84/38

(84) Designated Contracting States: CH DE FR GB IT LI

(71) Applicant: SUMITOMO CHEMICAL COMPANY, LIMITED
15 Kitahama 5-chome Higashi-ku
Osaka-shi Osaka-fu(JP)

(72) Inventor: Kondo, Michitada
2-14-7, Mefu
Takarazuka-shi Hyogo 665(JP)

(72) Inventor: Sato, Ryo
2-10-3-334, Sonehigashimachi
Toyonaka-shi Osaka 561(JP)

(72) Inventor: Matsumoto, Hiroshi
2-10-2-211, Sonehigashimachi
Toyonaka-shi Osaka 561(JP)

(72) Inventor: Okabe, Takayuki
4-2-202, Ryodocho
Nishinomiya-shi Hyogo 662(JP)

(74) Representative: Myerscough, Philip Boyd et al,
J.A.Kemp & Co. 14, South Square Gray's Inn
London, WC1R 5EU(GB)

## (54) Organic phosphorous quinoxalinone and their production and use.

(57) Organic phosphorous quinoxalinones of the formula:

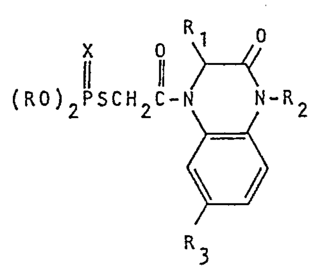

wherein R is a $C_1$ - $C_4$ alkyl group; $R_1$ and $R_2$ are the same or different $C_1$ - $C_4$ alkyl groups or hydrogen atoms; $R_3$ is a hydrogen, a chlorine or a fluorine atom or a $C_1$ - $C_4$ alkyl group; X is an oxygen or a sulfur atom, which is useful as a herbicide.

EP 0 118 982 A1

# ORGANIC PHOSPHOROUS QUINOXALINONE AND THEIR PRODUCTION AND USE

## General Statement of the Invention

The present invention relates to organic phosphorous quinoxalinone(s) and their production and use.

Said organic phosphorous quinoxalinones are representable by the formula:

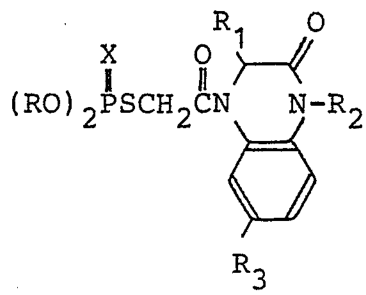

[I]

wherein R is a $C_1$ - $C_4$ alkyl group; $R_1$ and $R_2$ are each the same or different $C_1$ - $C_4$ alkyl groups or hydrogen atoms; $R_3$ is a hydrogen, a chlorine, or a fluorine atom or a $C_1$ - $C_4$ alkyl group; X is an oxygen or a sulfur atom.

Preferred are those [I] wherein R is a $C_1$ - $C_3$ alkyl group; $R_1$ is a hydrogen atom or a methyl group; $R_2$ is a hydrogen atom or a $C_1$ - $C_3$ alkyl group; $R_3$ is a hydrogen, a fluorine, or a chlorine atom, or a methyl group; and X is an oxygen or a sulfur atom.

An organic phosphorous quinoxalinone [I] of this invention is effective as herbicide to various sorts of weeds which will grow to disturb foliar or soil treatment on plowed field for agricultural crops and it exerts no material phytotoxicity on agriculturally cultivating plants as mentioned below.

| Japanese | English | Scientific name |
|---|---|---|
| Toomorokoshi | Corn, (Maize) | Zea mays |
| Komugi | Wheat | Triticum aestivum |
| Ine | Rice | Oryza sativa |
| Daizu | Soybean | Glycine max |
| Wata | Cotton | Gossypium sp. |
| Tensai | Sugar beet | Beta vulgaris |

Listed below are examples of weeds which the organic phosphorous quinoxalinones exert material herbicidal activity.

Weeds on Plowed Field

Broad-leafed weeds

| Japanese | English | Scientific name |
|---|---|---|
| Sobakazura | Wild buckwheat | Polygonum convolvulus |
| Suberihiyu | Common purslane | Portulaca oleracea |
| Hakobe | Chickweed | Stellaria media |
| Shiroza | Common lambsquarters | Chenopodium album |
| Aobiyu (hosoaogeitoo) | Pigweed | Amaranthus patulus |
| Daikon | Radish | Raphanus sativus |
| Noharagarashi | Wild mustard | Brassica kaber |
| Amerika-tsunokusanemu | Hemp sesbania | Sesbania exaltata |
| Ebisugusa | Sicklepod | Cassia obtusifolia |
| Ichibi | Velvetleaf | Abutilon theophrasti |
| Amerikakingojika | Prickly sida | Sida spinosa |
| Noraninjin | Wild carrot | Daucus carota |
| Amerikaasagao | Ivyleaf morningglory | Ipomoea hederacea |
| Marubaasagao | Tall morningglory | Ipomoea purpurea |
| Seiyoohirugao | Field bindweed | Convolvulus arvensis |
| Yooshuchoosen-asagao | Jimsonweed | Datura stramonium |
| Inuhoozuki | Black nightshade | Solanum nigrum |
| Inukamitsure | Scentless chamomile | Matricaria inodora |

Gramineous weeds

| | | |
|---|---|---|
| Hie | Japanese millet | Echinochloa frumentacea |
| Inubie | Barnyardgrass | Echinochloa crus-galli |
| Enokorogusa | Green foxtail | Setaria viridis |
| Mehishiba | Large crabgrass | Digitaria sanguinalis |
| Suzumenokatabira | Annual bluegrass | Poa annua |
| Nosuzumenoteppo | Black grass | Alopecurus myosuroides |
| Enbaku | Oat | Avena sativa |
| Karasumugi | Wild oat | Avena fatua |
| Seibanmorokoshi | Johnsongrass | Sorghum halepense |
| Shibamugi | Quackgrass | Agropyron repens |
| Umanochahiki | Downy Brome | Bromus tectorum |
| Gyoogishiba | Bermudagrass | Cynodon dactylon |
| Ohishiba | Goose grass | Eleusine indica |
| Commelina weeds | | |
| Tsuyukusa | Dayflower | Commelina communis |
| Sedge weeds | | |
| Kogomegayatsuri | Rice flatsedge | Cyperus iria |
| Hamasuge | Purple nutsedge | Cyperus rotundus |

Likewise, organic phosphorous quinoxalinones [I] of this invention exert herbicidal effects to weeds as listed below which will grow to disturb flood fallowing treatment in paddy field and those of this invention exert no material phytotoxicity on rice plant.

Weeds on Paddy Field

| | | |
|---|---|---|
| Gramineous weeds | | |
| Tainubie | Barnyardgrass | Echinochloa oryzicola |
| Broad-leafed weeds | | |
| Azena | False pimpernel | Lindernia procumbens |
| Kikashigusa | Redstem, rotala | Rotata indica |
| Mizohakobe | Waterwort | Elatine triandra |
| Sedge weeds | | |
| Tamagayatsuri | Umbrellaplant | Cyperus defformis |
| Hotarui | Hardstem bulrush | Scirpus juncoides |

| | | |
|---|---|---|
| Matsubai | Slender spikerush | Eleocharis acicularis |
| Mizugayatsuri | Water nutgrasse | Cyperus serotinus |
| Konagi | Pickerelweed (ducktongue weed) | Monochoria vaginalis |

Accordingly, organic phosphorous quinoxalinones [I] can be used as herbicide applicable to agricultural plowed field as well as paddy field. They are likewise useful as herbicide to be employed for orchard, pasture land, lawn, forest, non-agricultural field, etc.

Production of Compounds

An organic phosphorous quinoxalinone [I] of this invention is obtainable by a reaction between the following two kinds of compounds [II] and [III].

A compound [II] is (di)thiophosphonate representable by the formula:

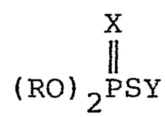

[II]

wherein R and X are each as defined above, and Y is an alkali metal atom or an ammonium group.

A compound [III] is haloacetyl quinoxalinone representable by the formula:

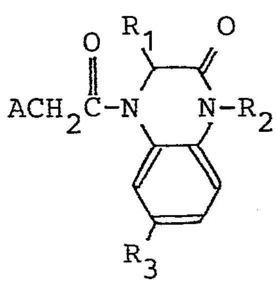

[III]

wherein A is a chlorine or a bromine atom, $R_1$, $R_2$ and $R_3$ are as defined above [I].

The reaction above is usually carried out in a solvent at a temperature of from 10 to 100°C for the period of from 0.5 to 24 hours. The compound [III] is normally employed in an amount of 0.8 to 1 equivalent to the compound [II].

Examples of the solvent are aliphatic hydrocarbons e.g. hexane, heptane, ligroin, petroleum ether, aromatic hydrocarbons e.g. benzene, toluene, xylene, halogenated hydrocarbons e.g. chloroform, carbon tetrachloride, dichloroethane, chlorobenzene, dichlorobenzene, ethers e.g. diethyl ether, diisopropyl ether, dioxane, tetrahydrofuran, diethyleneglycol dimethyl ether, ketones e.g. acetone,methylethyl ketone, methylisobutyl ketone, isophorone, cyclohexanone, alcohols e.g. methanol, ethanol, isopropanol, t-butanol, octanol, cyclohexanol, methylcellosolve, diethyleneglycol, glycerine, esters e.g. ethyl formate, ethyl acetate, butyl acetate, diethyl carbonate, nitro compounds e.g. nitroethane, nitrobenzene, nitriles e.g. acetonitrile, isobutyl nitrile, amides e.g. formamide, N,N-dimethylformamide, N,N-dimethylacetoamide, sulfuric compounds e.g. dimethyl sulfoxide, sulfolane, water or mixture thereof.

After the reaction, the product is subjected to such a routine post-treatment as extraction with an organic solvent and/or concentration, and, when desired, purified by per se conventional procedure such as chromatography, distillation or recrystallization.

Practical and presently preferred embodiments of the production of organic phosphorous quinoxalinones [I] are illustratively shown below:

Production Example 1

2.39 g of 1-methyl-4-chloroacetyl-1,2,3,4-tetrahydro-2-quinoxalinone was dissolved into 50 ml of acetone

and thereto 2.03 g of ammonium diethyldithiophosphate was added at once. Then the reaction mixture was stirred at a room temperature for 12 hours. The resultant liquid was put into 100 ml of toluene and was washed with water. Thereafter the organic layer was dried with dried magnesium sulfate and concentrated under reduced pressure. Thereby obtained was 3.8 g of needle-like crystalline 4-(O,O-diethyldithiophosphorylacetyl)-1-methyl-1,2,3,4-tetrahydro-2-quinoxalinone (Compound No. 3) of the melting point 119.6°C. 98 % yield.

Production Example 2

0.3 g of 1-methyl-4-(chloroacetyl)-6-chloro-1,2,3,4-tetrahydro-2-quinoxalinone was dissolved into 50ml of acetone and thereto 0.25 g of ammonium diethyldithiophosphate was added at once. Then the reaction mixture was stirred at a room temperature for 12 hours. The resultant liquid was put into 100 ml of toluene and washed with water. Thereafter the organic layer was dried with dried magnesium sulfate and concentrated under reduced pressure. Thereby obtained was 0.53 g of yellowish brown crystal, which was subjected to the purification by a column chromatograph packed with silica gel wherein elusion liquid was toluene/ethylacetate. Resultantly, obtained was 0.43 g of 4-(O,O-diethyldithiophosphorylacetyl)-1-methyl-6-chloro-1,2,3,4-tetrahydro-2-quinoxalinone (Compound No. 27) of the melting point 114.2°C, yellowish white needle-like crystal. 98 % yield.

Examples of the organic phosphorous quinoxalinone [I] produced in the same manner as above are shown in Table 1.

Table 1

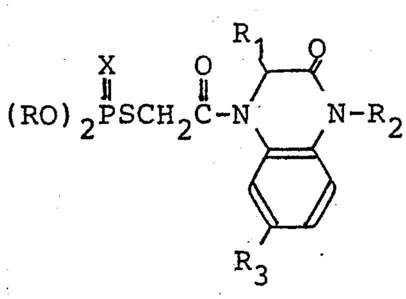

[I]

| Com'd No. | R | $R_1$ | $R_2$ | $R_3$ | X | Physical properties |
|---|---|---|---|---|---|---|
| 1 | $CH_3$ | H | $CH_3$ | H | S | m.p. 102.9°C |
| 2 | $CH_3$ | H | $CH_3$ | H | O | m.p. 116.5°C |
| 3 | $C_2H_5$ | H | $CH_3$ | H | S | m.p. 119.6°C |
| 4 | $C_2H_5$ | H | $CH_3$ | H | O | m.p. 102 °C |
| 5 | n-$C_3H_7$ | H | $CH_3$ | H | S | m.p. 120.5°C |
| 6 | iso-$C_3H_7$ | H | $CH_3$ | H | S | m.p. 126.5°C |
| 7 | $CH_3$ | H | $C_2H_5$ | H | S | m.p. 77.5°C |
| 8 | $CH_3$ | H | $C_2H_5$ | H | O | m.p. 133.4°C |
| 9 | $C_2H_5$ | H | $C_2H_5$ | H | S | m.p. 112.3°C |
| 10 | $C_2H_5$ | H | $C_2H_5$ | H | O | m.p. 102.9°C |
| 11 | n-$C_3H_7$ | H | $C_2H_5$ | H | S | m.p. 113 °C |
| 12 | iso-$C_3H_7$ | H | $C_2H_5$ | H | S | m.p. 187 °C |
| 13 | $CH_3$ | H | n-$C_3H_7$ | H | S | m.p. 68.1°C |
| 14 | $CH_3$ | H | n-$C_3H_7$ | H | O | m.p. 105.3°C |
| 15 | $C_2H_5$ | H | n-$C_3H_7$ | H | S | m.p. 117.2°C |
| 16 | $C_2H_5$ | H | n-$C_3H_7$ | H | O | m.p. 97.4°C |
| 17 | n-$C_3H_7$ | H | n-$C_3H_7$ | H | S | m.p. 94.3°C |
| 18 | iso-$C_3H_7$ | H | n-$C_3H_7$ | H | S | m.p. 112.7°C |
| 19 | $C_2H_5$ | H | iso-$C_3H_7$ | H | S | m.p. 125.3°C |
| 20 | $C_2H_5$ | H | iso-$C_3H_7$ | H | O | m.p. 136.1°C |
| 21 | n-$C_3H_7$ | H | iso-$C_3H_7$ | H | S | m.p. 108.9°C |
| 22 | $C_2H_5$ | $CH_3$ | $CH_3$ | H | S | $n_D^{24}$ 1.5790 |
| 23 | $C_2H_5$ | $CH_3$ | $CH_3$ | H | O | $n_D^{23.5}$ 1.5506 |
| 24 | $C_2H_5$ | H | H | Cl | S | m.p. 119.3°C |

(Table 1 continued)

| | | | | | | |
|---|---|---|---|---|---|---|
| 25 | $CH_3$ | H | $CH_3$ | Cl | S | $n_D^{26}$ 1.5670 |
| 26 | $CH_3$ | H | $CH_3$ | Cl | O | m.p. 123.6°C |
| 27 | $C_2H_5$ | H | $CH_3$ | Cl | S | m.p. 114.2°C |
| 28 | $C_2H_5$ | H | $CH_3$ | Cl | O | m.p. 118.2°C |
| 29 | n-$C_3H_7$ | H | $CH_3$ | Cl | S | m.p. 72.6°C |
| 30 | iso-$C_3H_7$ | H | $CH_3$ | Cl | S | $n_D^{25}$ 1.5766 |
| 31 | $CH_3$ | H | $C_2H_5$ | Cl | S | $n_D^{26}$ 1.5940 |
| 32 | $CH_3$ | H | $C_2H_5$ | Cl | O | m.p. 129 °C |
| 33 | $C_2H_5$ | H | $C_2H_5$ | Cl | S | m.p. 102.4°C |
| 34 | $C_2H_5$ | H | $C_2H_5$ | Cl | O | m.p. 107.8°C |
| 35 | n-$C_3H_7$ | H | $C_2H_5$ | Cl | S | m.p. 77.1°C |
| 36 | $C_2H_5$ | H | H | F | S | m.p. 119.3°C |
| 37 | $C_2H_5$ | H | $CH_3$ | F | S | m.p. 143.8°C |
| 38 | $C_2H_5$ | H | $CH_3$ | F | O | m.p. 116.3°C |
| 39 | n-$C_3H_7$ | H | $CH_3$ | F | S | m.p. 121 °C |
| 40 | $C_2H_5$ | H | $C_2H_5$ | F | S | m.p. 107.8°C |
| 41 | $C_2H_5$ | H | $C_2H_5$ | F | O | m.p. 118.3°C |
| 42 | $C_2H_5$ | H | $C_2H_5$ | F | S | m.p. 124.5°C |
| 43 | $C_2H_5$ | H | $CH_3$ | $CH_3$ | S | $n_D^{28}$ 1.5752 |
| 44 | $C_2H_5$ | H | $CH_3$ | $CH_3$ | O | m.p. 70.5°C |
| 45 | $C_2H_5$ | H | $C_2H_5$ | $CH_3$ | S | m.p. 61 °C |
| 46 | $C_2H_5$ | H | $C_2H_5$ | $CH_3$ | O | m.p. 67 °C |
| 47 | n-$C_3H_7$ | H | $C_2H_5$ | $CH_3$ | S | m.p. 75 °C |

The material compound [III], haloactyl quinoxalinone, to produce the organic phosphorous quinoxalinone [I] of this invention is obtainable by a reaction between a compound [IV], and a compound [V]:

The compound [IV] is quinoxalinone representable by the formula:

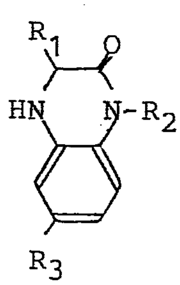

[IV]

wherein $R_1$, $R_2$ and $R_3$ are as defined above.

The compound [V] is haloacetyl halide representable by the formula:

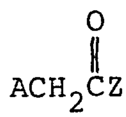

[V]

wherein A and Z are each the same or different halogen atoms.

The reaction above is usually carried out in a solvent in the presence of an organic base at a temperature of from 0 to 50°C for a period of from 2 to 6 hours.

The compound [V] is normally employed in an amount of 1 to 3 equivalents to the compound [IV]. The amount of the base may be ordinarily from 1 to 3 equivalents to the compound [IV].

Example of the solvent are halogenated hydrocarbon e.g. chloroform, dichloromethane, dichloroethane or aromatic hydrocarbon e.g. benzene, toluene, xylene. As the base, there may be exemplified pyridine, triethylamine, N,N-diethylaniline, tributylamine, etc.

After the reaction, the product is subjected to such a routine post-treatment, and when desired, purified by a per se conventional procedure such as chromatography or recrystallization.

Typical examples for the production of the compound [III] are illustratively shown below.

<u>Production Example 3</u>

16.2 g of 1-methyl-1,2,3,4-tetrahydro-2-quinoxalinone was dissolved in 300 ml of chloroform and thereto 33.9 g of chloroacetylchloride was added at once. Therein

30.4 g of triethylamine was instilled under stirring at 0 - 5°C and thereafter stirring was continued at a room temperature for 3 hours. The resultant liquid was washed with 1-N HCl, water, saturated sodium bicarbonate aq., saturated NaCl aq. in sequence and then dried by dried magnesium sulfate. The organic layer was concentrated under reduced pressure to yield 25 g of crude crystal, which was recrystallized with 95 % ethanol. Thereby obtained was 21.5 g of 1-methyl-4-chloroacetyl-1,2,3,4-tetrahydro-2-quinoxalinone of the melting point 176.2°C and needle-like crystal. 90 % yield.

Production Example 4

1.22 g of 1-methyl-6-chloro-1,2,3,4-tetrahydro-2-quinoxalinone was dissolved in 50 ml of chloroform and thereto 1.05 g of chloroacetylchloride was added at once. Therein 0.94 g of triethylamine was instilled under stirring at 0 - 5°C and thereafter stirring was continued at a room temperature for 3 hours. The resultant liquid was washed with 1-N HCl, saturated sodium bicarbonate, water in sequence and then dried by dried magnesium sulfate. The organic layer was concentrated under reduced pressure to yield 2.2 g of yellowish-brown crystal, which was purified by the column chromatograph packed with silica gel wherein elution liquid was toluene/ethyl acetate. Thereby obtained was 1.53 g of 1-methyl-4-chloroacetyl-6-chloro-1,2,3,4-tetrahydro-2-quinoxalinone of the melting point 112°C and yellow needle-like crystal. 90 % yield.

Formulation of Herbicide

An organic phosphorous quinoxalinone [I] of this invention is formulated into a herbicidal composition so that the organic phosphorous quinoxalinone [I] is contained as active ingredient therein in the range of 0.1 - 90 % by weight, preferably 0.2 - 80 % and that the remaining part is shared by inert carrier or diluent e.g. solid carrier,

liquid carrier, surface active agent and other formulation adjuvants and may be formulated in any composition form of emulsifiable concentrate, wettable powder, suspension, granule, etc.

Examples of the solid carrier or diluent are kaolin clay, attapulgite clay, bentonite, terra alba, pyrophyllite, talc, diatomaceous silica, calcite, walnut powder, urea, ammonium sulfate, synthetic hydrated silicon dioxide and like fine powders or particles. As the liquid carrier or diluent, there may be exemplified aromatic hydrocarbons,e.g. xylene, methylnaphthalene, alcohols, e.g. isopropanol, ethylene glycol, cellosolve, ketones, e.g. acetone, cyclohexanone, isophorone, vegetable oils, e.g. soy bean oil, cotton seed oil, dimethyl sulfoxide, acetonitrile and water.

The surface active agent used for emulsification, dispersion or spreading may be any of the anionic and nonionic type of agents. Examples thereof include; alkylsulfates, alkylarylsulfonates, dialkylsulfosuccinates, phosphates of polyoxyethylenealkylaryl ethers, polyoxyethylene alkyl ethers, polyoxyethylene alkylaryl ethers, polyoxyethylene polyoxypropylene block copolymer, sorbitan fatty acid esters, polyoxyethylene sorbitan fatty acid esters, etc. Examples of formulation adjuvants include; ligninsulfonates, sodium alginate, polyvinyl alcohol, gum arabic, CMC or carboxymethyl cellulose, PAP or isopropyl acid phospate, etc.

Practical embodiments of the herbicidal composition according to the invention are illustratively shown in the following examples wherein parts are by weight. The compound number of the active ingredient corresponds to the one in Table 1.

Formulation Example 1

50 parts of the compound No. 3 or No. 43 as active ingredient, 3 parts of calcium ligninsulfonate, 2 parts of sodium laurylsulfate and 45 parts of synthetic hydrated silicon dioxide are well mixed while being powdered to obtain a wettable powder.

Formulation Example 2

2 parts of the compound No. 10 or No. 37, 14 parts of polyoxyethylenestyryl phenyl ether, 6 parts of calcium dodecylbenzenesulfonate and 30 parts of xylene and 48 parts of isophorone are well mixed while being powdered to obtain an emulsifiable concentrate.

Formulation Example 3

2 parts of the compound No. 9 or No. 27, 1 part of synthetic hydrated silicon dioxide, 2 parts of calcium ligninsulfonate, 30 parts of bentonite and 65 parts of kaolin clay are well mixed while being powdered. The mixture is then kneaded with water, granulated and dried to obtain granules.

Formulation Example 4

25 parts of the compound No. 9 or No. 34, 3 parts of polyoxyethylene sorbitan monooleate, 3 parts of CMC and 69 parts of water are well mixed and pulverized until the particle size of powders becomes less than 5 microns to obtain a suspension.

The organic phosphorous quinoxalinone [I] thus formulated in any suitable composition form is useful for the pre- or post-germination control of undesired weeds by means of soil or foliar treatment as well as flood fallowing treatment. These treatments include the application to the soil surface prior or subsequent to planting, and the incorporation into the soil. The foliar treatment may

be effected by spraying the herbicidal composition over the top of plants. Otherwise it may be applied directly to weeds if good care is taken to keep the chemical off the crop foliage.

The herbicidal composition may be used in mixture or blend of some other herbicide to improve their activity, and in some cases, a synergistic effect can be expected. Further, they may be applied in combination with insecticides, acaricides, nematocides, fungicides, plant growth regulators, fertilizers, soil improvers, etc.

Use as Herbicides

The dosage rate of an organic phosphorous quinoxalinone [I] of this invention as active ingredient in a herbicide may vary depending on prevailing weather conditions, site condition, preparation used, prevailing season, mode of application, soil involved, crop and weed species, etc. Generally, however, the dosage rate ranges for 0.5-200 g, preferably 1 - 100 g of the active ingredient per are. The herbicidal composition formulated in the form of an emulsifiable concentrate, a wettable powder or a suspension may ordinarily be employed by diluting it with water at a volume of 1 - 10 liters per are, if necessary, with addition of an adjuvant such as a spreading agent. Examples of the spreading agent include, in addition to the surface active agents as noted above; polyoxyethylene resin acid ester, ligninsulfonate, abiethylenic acid salt, dinaphthylmethane disulfonate, paraffin, etc. The herbicidal composition formulated in the form of granule may normally be applied as it is, without dilution.

In the following, the biological data of organic phosphorous quinoxalinones [I] as herbicide are illustratively shown in test examples, wherein an organic phosphorous quinoxalinone compound noted by the compound number listed in Table 1 and a control compound is by a mark letter noted in Table 2 below:

Table 2

| Mark | Formula of the control compound | Common name |
|---|---|---|
| A | 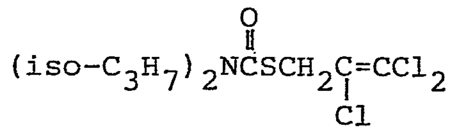 | Tri-allate |
| B | 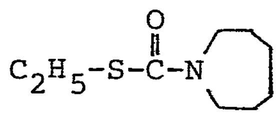 | Molinate |

The phytotoxicity to crop plants and the herbicidal activity on weeds were observed visually in terms of the degree of germination as well as the growth inhibition and rated with a ranking index in any of 0, 1, 2, 3, 4 or 5, in which the numeral "0" indicates that no material difference is observed in comparison with the untreated plant and the numeral "5" indicates the complete inhibition or death of a test plant.

Test Example 1

Soil treatment, admixing into soil of plowed field.

Soil of plowed field was filled in cylindrical plastic pots wherein diameter-10 cm; height-10 cm and thereon seeds of Japanese millet (Hie), Wild oat (Karasumugi), Large crabgrass (Mehishiba), Green foxtail (Enokorogusa) were sowed and covered with soil.

Then a designed amount of the test compound, which had been formulated into an emulsifiable concentrate according to Formulation Example 2 and diluted with water comparable to a volume of 10 liters per are, was sprayed by

a small sprayer over the surface of the soil in pots. Thereafter the surface soil for 4 cm in depth was well blended. Test plants were cultivated in a greenhouse for 20 days subsequent to the soil treatment above while the herbicidal activity was examined. Results are shown in Table 3.

Table 3

| Test compound | Dosage of active ingredient (g/a) | Herbicidal activity | | | |
|---|---|---|---|---|---|
| | | Japanese millet | Wild oat | Large crabgrass | Green foxtail |
| 1 | 20 | 5 | 4 | 5 | 5 |
| 2 | 20 | 5 | 4 | 5 | 5 |
| 3 | 20<br>5 | 5<br>5 | 5<br>- | 5<br>5 | 5<br>5 |
| 4 | 20<br>5 | 5<br>5 | 5<br>4 | 5<br>5 | 5<br>5 |
| 5 | 20<br>5 | 5<br>4 | 4<br>4 | 5<br>5 | 5<br>4 |
| 7 | 20 | 5 | - | 5 | 5 |
| 8 | 20 | 5 | 4 | 5 | 5 |
| 9 | 20<br>5 | 5<br>4 | 4<br>- | 5<br>5 | 5<br>5 |
| 10 | 20<br>5 | 5<br>5 | 5<br>- | 5<br>4 | 5<br>4 |
| 11 | 20 | 4 | - | 5 | 5 |
| 12 | 20 | 4 | - | 4 | 4 |
| 15 | 20 | 5 | - | 5 | 5 |
| 16 | 20 | 5 | 4 | 5 | 5 |
| 20 | 20 | 4 | 4 | 5 | 4 |
| 22 | 20<br>5 | 5<br>4 | 4<br>- | 5<br>4 | 5<br>5 |

(Table 3 continued)

| | | | | | |
|---|---|---|---|---|---|
| 23 | 20<br>5 | 5<br>4 | 4<br>– | 5<br>5 | 5<br>4 |
| 25 | 20 | 5 | 4 | 5 | 5 |
| 26 | 20 | 5 | 5 | 5 | 5 |
| 27 | 20<br>5 | 5<br>5 | 5<br>– | 5<br>5 | 5<br>5 |
| 28 | 20<br>5 | 5<br>5 | 5<br>– | 5<br>5 | 5<br>5 |
| 29 | 20 | 5 | 5 | 5 | 5 |
| 31 | 20 | 5 | – | 5 | 5 |
| 32 | 20 | 5 | – | 5 | 5 |
| 33 | 20<br>5 | 5<br>5 | 5<br>– | 5<br>4 | 5<br>5 |
| 34 | 20<br>5 | 5<br>4 | 4<br>– | 5<br>5 | 5<br>5 |
| 35 | 20 | 4 | – | 4 | 5 |
| 36 | 20 | 5 | – | 5 | 5 |
| 37 | 20<br>5 | 5<br>5 | 5<br>– | 5<br>5 | 5<br>5 |
| 38 | 20<br>5 | 5<br>5 | 5<br>– | 5<br>5 | 5<br>5 |
| 39 | 20 | 5 | 5 | 5 | 5 |
| 40 | 20<br>5 | 5<br>5 | 5<br>– | 5<br>4 | 5<br>4 |
| 41 | 20<br>5 | 5<br>5 | 5<br>– | 5<br>4 | 5<br>4 |
| 42 | 20 | 4 | – | 4 | 4 |
| 43 | 20<br>5 | 5<br>4 | 4<br>– | 5<br>4 | 5<br>4 |
| 44 | 20<br>5 | 5<br>4 | 5<br>– | 5<br>4 | 5<br>4 |
| 45 | 20 | 5 | 4 | 5 | 5 |
| 46 | 20 | 5 | 4 | 5 | 5 |

(Table 3 continued)

| | | | | | |
|---|---|---|---|---|---|
| 47 | 20 | 4 | - | 4 | 4 |
| A | 20<br>5 | 2<br>0 | 3<br>1 | 2<br>0 | 3<br>1 |

Test Example 2

Foliar treatment, plowed field.

Cylindrical plastic pots wherein diameter-10 cm, height-10 cm were filled with soil of plowed field and seeds of Japanese millet (Hie), Oat (Enbaku), Radish (Daikon), Velvetleaf (Ichibi) were sowed and cultivated for 10 days in a greenhouse. Thereafter a designed amount of the test compound, which had been formulated into an emulsifiable concentrate according to Formulation Example 2 and diluted with water containing a spreading agent comparable to 10 liters per are, was sprayed by a small sprayer over the top of weeds to effect the foliar treatment. Treated weeds were cultivated in a greenhouse for further 20 days subsequent to the treatment while the herbicidal activity was examined. Results are shown in Table 4.

Table 4

| Test compound | Dosage of active ingredient (g/a) | Herbicidal activity | | | |
|---|---|---|---|---|---|
| | | Japanese millet | Oat | Radish | Velvetleaf |
| 8 | 80 | 5 | 4 | 4 | 5 |
| 9 | 40 | 5 | 4 | 4 | 5 |
| 10 | 80 | 5 | 4 | 4 | 5 |
| 11 | 80 | 4 | - | 4 | 4 |
| 16 | 80 | 5 | 4 | 4 | 4 |
| 22 | 80 | 5 | - | 4 | 4 |
| 27 | 80 | 5 | 5 | 5 | 5 |
| 28 | 40 | 5 | 4 | 4 | 5 |
| 29 | 80 | 5 | 5 | 5 | 5 |

(Table 4 continued)

| | | | | | |
|---|---|---|---|---|---|
| 33 | 80 | 5 | 4 | 4 | 5 |
| 34 | 40 | 5 | 4 | 4 | 4 |
| 35 | 80 | 5 | 4 | 4 | 5 |
| 36 | 80 | 4 | 4 | 4 | 4 |
| 37 | 40 | 5 | 4 | 5 | 5 |
| 38 | 40 | 5 | 5 | 5 | 5 |
| 39 | 80 | 5 | 4 | 5 | 5 |
| 40 | 80 | 5 | 4 | 4 | 5 |
| 41 | 40 | 5 | 4 | 4 | 4 |
| 42 | 80 | 5 | 4 | 4 | 5 |
| 43 | 80 | 5 | 4 | 4 | 4 |
| 44 | 80 | 5 | 5 | 4 | 5 |
| 45 | 80 | 5 | 4 | - | 4 |
| 46 | 80 | 5 | 4 | 4 | 4 |
| 47 | 80 | 4 | 4 | - | 4 |

Test Example 3

Flood fallowing treatment, paddy field.

Cylindrical plastic pots wherein diameter-8 cm, height-12 cm were filled with soil of paddy field and seeds of Barnyardgrass (Tainubie), three spacies of broad-leafed [False pimpernel (Azena), Redstem rotala (Kikashigusa), Waterwart (Mizohakobe)] and Hardstem bulrush (Hotarui) were sowed in 1 - 2 cm depth. Then the pots were flooded like paddy field. Thereafter rice seedlings at two-leaf stage were transplanted and cultivated in a greenhouse. 6 days afterward when weeds were at their initial germination, a designed amount of the test compound, which had been formulated in an emulsifiable concentrate according to Formulation Example 2 and diluted with 5 milli liter of water, was applied on water surface. Subsequent thereto, plants were cultivated in a greenhouse for further 20 days while the herbicidal activity was examined. Results are shown in Table 5.

Table 5

| Test compound | Dosage of active ingredient (g/a) | Herbicidal activity | | | |
|---|---|---|---|---|---|
| | | Rice | Barnyard-grass | Broad-leafed weeds | Hardstem bulrush |
| 1 | 10 | 0 | 5 | 5 | 4 |
| 3 | 5<br>2.5 | -<br>- | 5<br>5 | 5<br>4 | 4<br>4 |
| 4 | 5<br>2.5 | 1<br>0 | 5<br>5 | 5<br>4 | 4<br>4 |
| 5 | 5<br>2.5 | 0<br>0 | 5<br>5 | 4<br>4 | 4<br>4 |
| 6 | 10<br>5 | 0<br>0 | 5<br>5 | 5<br>4 | 4<br>4 |
| 7 | 5<br>2.5 | 0<br>0 | 5<br>5 | 4<br>4 | 4<br>4 |
| 9 | 5<br>2.5 | 0<br>0 | 5<br>5 | 5<br>4 | 4<br>4 |
| 10 | 10<br>5 | 1<br>0 | 5<br>5 | 4<br>4 | 4<br>4 |
| 11 | 5<br>2.5 | 0<br>0 | 5<br>5 | 5<br>4 | 4<br>4 |
| 12 | 5<br>2.5 | 0<br>0 | 5<br>5 | 5<br>- | 4<br>- |
| 13 | 10 | 0 | 5 | 4 | 4 |
| 15 | 5<br>2.5 | 0<br>0 | 5<br>5 | 5<br>4 | 4<br>4 |
| 16 | 10 | 0 | 5 | 5 | 4 |
| 17 | 10<br>5 | 0<br>0 | 5<br>4 | 4<br>4 | 4<br>4 |
| 19 | 5<br>2.5 | 0<br>0 | 5<br>5 | 4<br>- | -<br>- |
| 20 | 10 | 0 | 5 | 5 | - |

(Table 5 continued)

| | | | | | |
|---|---|---|---|---|---|
| 21 | 10<br>5 | 0<br>0 | 5<br>4 | 4<br>– | –<br>– |
| 22 | 5<br>2.5 | 0<br>0 | 5<br>5 | 4<br>4 | 4<br>– |
| 24 | 10 | 0 | 5 | 4 | – |
| 25 | 10 | 0 | 5 | 5 | 4 |
| 27 | 5<br>2.5 | 1<br>0 | 5<br>5 | 5<br>5 | 5<br>4 |
| 28 | 5<br>2.5 | –<br>0 | 5<br>5 | 5<br>4 | 4<br>– |
| 29 | 5<br>2.5 | 0<br>0 | 5<br>5 | 4<br>4 | 4<br>– |
| 30 | 10 | 0 | 5 | 5 | 4 |
| 31 | 10<br>5 | –<br>0 | 5<br>5 | 5<br>5 | 4<br>4 |
| 33 | 5<br>2.5 | 1<br>0 | 5<br>5 | 5<br>4 | 4<br>– |
| 34 | 5<br>2.5 | 1<br>1 | 5<br>5 | 4<br>4 | –<br>– |
| 35 | 5 | 0 | 5 | 5 | – |
| 36 | 10 | 0 | 5 | 4 | – |
| 37 | 5<br>2.5 | 0<br>0 | 5<br>5 | 5<br>5 | 5<br>– |
| 39 | 5<br>2.5 | 0<br>0 | 5<br>4 | 5<br>4 | 4<br>– |
| 40 | 5<br>2.5 | 0<br>0 | 5<br>5 | 4<br>4 | 4<br>– |
| 41 | 5 | – | 5 | 5 | 4 |
| 42 | 5<br>2.5 | 0<br>0 | 5<br>5 | 5<br>4 | 4<br>– |
| 43 | 5<br>2.5 | 1<br>0 | 5<br>5 | 5<br>5 | 4<br>– |
| 44 | 5<br>2.5 | 1<br>0 | 5<br>5 | 5<br>5 | 4<br>– |

(Table 5 continued)

| | | | | | |
|---|---|---|---|---|---|
| 45 | 5 | 1 | 5 | 5 | 4 |
| 46 | 5 | 0 | 5 | 5 | - |
| 47 | 5 | 1 | 5 | 5 | - |
| B | 10<br>5<br>2.5 | 0<br>0<br>0 | 4<br>3<br>1 | 2<br>1<br>0 | 3<br>2<br>0 |

Test Example 4

Soil treatment, plowed field.

Vats measuring the area 33 x 23 $cm^2$ and the depth 11 cm were filled with soil of plowed field, where Corn (Toomorokoshi), Soybean (Daizu), Cotton (Wata), Goosegrass (Ohishiba), Johnsongrass (Seibanmorokoshi), Green foxtail (Enokorogusa), Barnyardgrass (Inubie), Pigweed (Aobiyu) were sowed and covered with soil in 1 - 2 cm depth. A designed amount of the test compound, which had been formulated into an emulsifiable concentrate according to Formulation Example 2 are diluted with water comparable to 10 liters per are, was applied to soil surface by a small sprayer. Thereafter, the test plants were cultivated in a greenhouse for 20 days while the herbicidal activity was examined. Results are shown in Table 6.

Table 6

| Test compound | Dosage of active ingredient (g/a) | Herbicidal activity | | | | | | | | |
|---|---|---|---|---|---|---|---|---|---|---|
| | | Corn | Soy-bean | Cotton | Goose-grass | Large crab-grass | Johnson-grass | Green foxtail | Barn-yard-grass | Pigweed |
| 3 | 6 | 2 | 1 | 0 | 5 | 5 | 5 | 5 | 5 | 5 |
| | 3 | 0 | 0 | 0 | 5 | 5 | 4 | 5 | 4 | 4 |
| 4 | 6 | 2 | 1 | 1 | 5 | 5 | 5 | 5 | 5 | 5 |
| | 3 | 0 | 0 | 0 | 5 | 4 | 4 | 4 | – | 4 |
| 9 | 6 | 1 | 0 | 0 | 5 | 5 | 5 | 4 | 4 | 5 |
| | 3 | 0 | 0 | 0 | 5 | 5 | 4 | – | – | – |
| 10 | 6 | 1 | 0 | 0 | 5 | 5 | 4 | 5 | 5 | 5 |
| | 3 | 0 | 0 | 0 | 4 | 4 | – | 4 | – | 4 |
| 22 | 6 | 2 | 1 | 0 | 5 | 5 | 5 | 5 | 4 | 5 |
| | 3 | 0 | 0 | 0 | 5 | 4 | – | – | – | – |
| 27 | 6 | 2 | 0 | 0 | 5 | 5 | 5 | 5 | 5 | 5 |
| | 3 | 0 | 0 | 0 | 5 | 5 | 4 | 5 | 4 | 4 |
| 28 | 6 | 1 | 0 | 0 | 5 | 5 | 5 | 5 | 5 | 5 |
| | 3 | 0 | 0 | 0 | 5 | 4 | 4 | 4 | 4 | 4 |
| 29 | 6 | 0 | 0 | 0 | 5 | 5 | 4 | 5 | 4 | 5 |
| 33 | 6 | 1 | 1 | 0 | 5 | 5 | 5 | 5 | 5 | 5 |
| | 3 | 0 | 0 | 0 | 5 | 4 | – | 4 | – | – |
| 34 | 6 | 1 | 0 | 0 | 5 | 5 | 5 | 5 | 5 | 5 |
| | 3 | 0 | 0 | 0 | 4 | 5 | – | 4 | 4 | 4 |

(Table 6 continued)

| | | | | | | | | | | |
|---|---|---|---|---|---|---|---|---|---|---|
| 37 | 6<br>3 | 2<br>0 | 0<br>0 | 0<br>0 | 5<br>5 | 5<br>5 | 5<br>4 | 5<br>5 | 5<br>4 | 5<br>5 |
| 38 | 6 | 1 | 0 | 0 | 5 | 5 | 4 | 5 | 4 | 5 |
| 39 | 6 | 0 | 0 | 0 | 5 | 5 | 4 | 4 | 4 | 4 |
| 41 | 6 | 1 | 0 | 0 | 5 | 5 | 4 | 4 | 4 | 4 |
| 43 | 6 | 1 | 0 | 0 | 5 | 5 | 4 | 5 | 4 | 5 |
| 44 | 6<br>3 | 1<br>0 | 0<br>0 | 0<br>0 | 5<br>4 | 5<br>4 | 5<br>– | 5<br>4 | 5<br>– | 5<br>4 |
| 46 | 6 | 1 | 0 | 0 | 5 | 4 | 4 | 5 | 4 | 4 |

Test Example 5

Flood fallowing treatment, paddy field.

Wagner's pots (1/5000 are) were filled with paddy field soil where seeds of Barnyardgrass (Tainubie), three species of broad-leafed [False pimpernel (Azena), Redstem rotala (Kikashigusa), Waterwort (Mizohakobe)], Hardstem bulrush (Hotarui) and winter-tided-over buds of Slender spikerush (Matsubai) were sowed in 1 - 2 cm depth. Water was poured therein to produce a flooded condition where rice seedlings at three leaf stage were transplanted. Then the test plants were cultivated in a greenhouse. Five days afterward when Barnyardgrass was at its initial germination, a designed amount of the test compound, which had been formulated into an emulsifiable concentrate according to Formulation Example 2 and diluted with 10 milli liter water, was applied to the water surface, thereby the depth thereof was controlled to be 4 cm. Subsequent to the application, the test plants were cultivated again in a greenhouse for further 20 days while the herbicidal activity was examined. In the meantime, for two days starting on the next day after the treatment, water was given at a volume comparable to 3 cm in water depth per day by the artificial leaching loss method. Results are shown in Table 7.

Table 7

| Test compound | Dosage of active ingredient (g/a) | Herbicidal activity | | | | |
|---|---|---|---|---|---|---|
| | | Rice | Barnyardgrass | Broad leafed weeds | Slender spikerush | Hardstem bulrush |
| 3 | 10 | 0 | 5 | 5 | 5 | 5 |
| | 5 | 0 | 5 | 5 | 5 | 4 |
| 5 | 10 | 0 | 5 | 5 | 5 | 4 |
| | 5 | 0 | 5 | 5 | 4 | - |
| 7 | 10 | 0 | 5 | 5 | 4 | 4 |

(Table 7 continued)

| | | | | | | |
|---|---|---|---|---|---|---|
| 9 | 10<br>5 | 0<br>0 | 5<br>5 | 5<br>5 | 5<br>4 | 4<br>– |
| 11 | 10<br>5 | 0<br>0 | 5<br>5 | 5<br>5 | 5<br>4 | 4<br>4 |
| 27 | 5<br>2.5 | 1<br>0 | 5<br>5 | 5<br>5 | 5<br>5 | 5<br>4 |
| 29 | 5<br>2.5 | –<br>0 | 5<br>5 | 5<br>5 | 5<br>5 | 5<br>– |
| 33 | 5<br>2.5 | 1<br>0 | 5<br>5 | 5<br>4 | 5<br>5 | 5<br>4 |
| 35 | 5<br>2.5 | 0<br>0 | 5<br>5 | 5<br>4 | 5<br>4 | 4<br>– |
| 37 | 5<br>2.5 | 0<br>0 | 5<br>5 | 5<br>5 | 5<br>5 | 5<br>– |
| 39 | 5<br>2.5 | 0<br>0 | 5<br>5 | 5<br>5 | 5<br>4 | 4<br>– |
| 40 | 5 | 0 | 5 | 5 | 5 | 4 |
| 42 | 5<br>2.5 | 0<br>0 | 5<br>5 | 5<br>4 | 5<br>4 | 4<br>– |
| 43 | 5<br>2.5 | 1<br>0 | 5<br>5 | 5<br>4 | 5<br>5 | 5<br>– |
| 44 | 5 | 1 | 5 | 5 | 4 | 4 |
| 45 | 5<br>2.5 | 1<br>0 | 5<br>4 | 5<br>4 | 5<br>4 | 4<br>– |
| B | 5<br>2.5 | 0<br>0 | 3<br>1 | 0<br>0 | 0<br>0 | 0<br>0 |

<u>Test Example 6</u>

Soil treatment, plowed field.

Vats, 33 x 23 x 11 cm, were filled with soil of plowed field, where seeds of wheat (Komugi), Wild oat (Karasumugi), Black grass (Nosuzumenoteppo), Annual blue-grass (Suzumenokatabira), Common lambsquarters (Shiroza),

Chickweed (Hakobe), Wild mustard (Noharagarashi) were sowed in 1 - 2 cm depth and covered with soil thereover. A designed amount of the test compound, which had been formulated into an emulsifiable concentrate according to Formulation Example 2 and diluted with water comparable to 10 liters per are, was sprayed by a small sprayer over soil surface. After the soil treatment, test plants were cultivated in a greenhouse for 28 days while the herbicidal activity was examined. Results are shown in Table 8.

Table 8

| Test compound | Dosage of active ingredient (g/a) | Herbicidal activity | | | | | | |
|---|---|---|---|---|---|---|---|---|
| | | Wheat | Wild vat | Black grass | Annual bluegrass | Common lambsquarters | Chick weed | Wild mustard |
| 3 | 10 | 2 | 4 | 5 | 5 | 5 | 4 | 4 |
| | 5 | 1 | – | 4 | 4 | 4 | – | – |
| 4 | 10 | 1 | 5 | 5 | 5 | 5 | 5 | 5 |
| | 5 | 0 | 4 | 4 | 5 | 5 | 4 | 4 |
| 27 | 10 | 1 | 5 | 5 | 5 | 5 | 5 | 5 |
| | 5 | 0 | – | 5 | 5 | 5 | 5 | 4 |
| 28 | 10 | 2 | 5 | 5 | 5 | 5 | 5 | 5 |
| | 5 | 0 | – | 5 | 5 | 5 | 4 | – |
| 37 | 10 | 2 | 5 | 5 | 5 | 5 | 5 | 4 |
| | 5 | 0 | – | 4 | 5 | 5 | – | – |
| 38 | 10 | 2 | 5 | 5 | 5 | 5 | 5 | 4 |
| | 5 | 0 | – | 4 | 5 | 5 | – | – |
| 39 | 10 | 1 | 4 | 4 | 5 | 4 | 4 | 4 |
| A | 10 | 0 | 3 | 3 | 2 | 1 | 0 | 0 |
| | 5 | 0 | 1 | 0 | 1 | 0 | 0 | 0 |

CLAIMS

1. An organic phosphorous quinoxalinone of the formula:

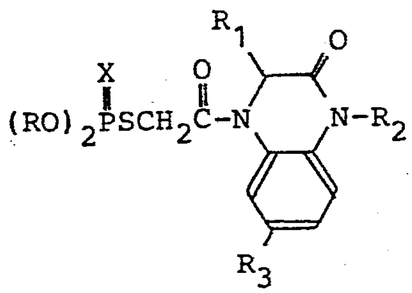

wherein R is a $C_1$-$C_4$ group, $R_1$ and $R_2$, which may be the same or different, each represent a $C_1$-$C_4$ alkyl group or hydrogen, $R_3$ is hydrogen, chlorine or fluorine or a $C_1$-$C_4$ alkyl group and X is oxygen or sulfur.

2. A compound according to claim 1, wherein R is a $C_1$-$C_3$ alkyl group, $R_1$ is hydrogen or methyl, $R_2$ is hydrogen or a $C_1$-$C_3$ alkyl group and $R_3$ is hydrogen, fluorine, chlorine or methyl.

3. 4-(O,O-Diethyldithiophosphorylacetyl)-1-methyl-1,2,3,4-tetrahydro-2-quinoxalinone.

4. 4-(O,O-Diethyldithiophosphorylacetyl)-1-methyl-6-chloro-1,2,3,4-tetrahydro-2-quinoxalinone.

5. 4-(O,O-Diethyldithiophosphorylacetyl)-1-methyl-6-fluoro-1,2,3,4-tetrahydro-2-quinoxalinone.

6. A process for producing a compound as claimed in claim 1, which comprises reacting a (di)thiophosphonate of the formula:

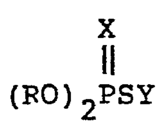

wherein R and X are as defined in claim 1, and Y is an alkali metal atom or an ammonium group, with a haloacetyl quinoxalinone of the formula:

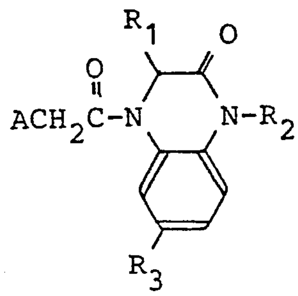

wherein A is chlorine or bromine and $R_1$, $R_2$ and $R_3$ are as defined in claim 1.

7. A herbicidal composition which comprises as active ingredient a herbicidally effective amount of a compound as claimed in any one of claims 1 to 5, and an inert carrier or diluent.

8. A method for controlling weeds which comprises applying to an area where weeds are growing or will grow a herbicidally effective amount of a compound as claimed in any one of claims 1 to 5 or of a composition as claimed in claim 7.

9. A method according to claim 8, wherein the area is an area where wheat, rice, or soybean is growing or will be grown.

10. A haloacetyl quinoxalinone of the formula:

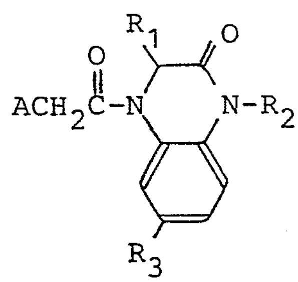

wherein A is a chlorine or a bromine atom; $R_1$, $R_2$ and $R_3$ are as defined in claim 1.

European Patent Office

**0118982**

Application number

# EUROPEAN SEARCH REPORT

EP 84 30 0532

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | US-A-4 225 595 (L.W. FANCHER)<br>* Column 1 *<br>--- | 1,6-10 | C 07 F 9/65<br>A 01 N 57/16<br>C 07 D 241/38 |
| A | DE-A-2 339 462 (SUMITOMO CHEMICAL CO.)<br>* Pages 1-3 *<br>----- | 1,6-10 | |
| | | | TECHNICAL FIELDS SEARCHED (Int. Cl. 3) |
| | | | C 07 F 9/00<br>C 07 D 241/00<br>A 01 N 57/00 |

The present search report has been drawn up for all claims

| Place of search | Date of completion of the search | Examiner |
|---|---|---|
| THE HAGUE | 27-04-1984 | BESLIER L.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document